# EUROPÄISCHE PATENTANMELDUNG

(11) **EP 4 710 877 A1**
(43) Veröffentlichungstag der Anmeldung: **18.03.2026**
(21) Anmeldenummer: 24199681.8
(22) Anmeldetag: 11.09.2024
(51) Int. Cl.: A61B 17/72

(54) **VORRICHTUNG UND VERFAHREN ZUR HERSTELLUNG EINES MARKNAGELSPACERS**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(57) **Zusammenfassung**

Die Erfindung betrifft eine Vorrichtung zur Herstellung eines Marknagelspacers, eine Verwendung einer Gussform sowie ein Verfahren zur Herstellung eines Marknagelspacers. Eine Vorrichtung (10) zur Herstellung eines Marknagelspacers (5) umfasst ein erstes Rohr (11), ein zweites Rohr (12) und ein flexibles Verbindungsstück (15) zum strömungstechnischen Verbinden des ersten Rohrs (11) mit dem zweiten Rohr (12), wobei das erste Rohr (11) und das zweite Rohr (12) in unterschiedlichen Winkeln zueinander positionierbar sind. Auf diese Weise können Marknagelspacer intraoperativ und mit erhöhter Genauigkeit hergestellt werden.

## Beschreibung

Die Erfindung betrifft eine Vorrichtung zur Herstellung eines Marknagelspacers, eine Verwendung einer Gussform sowie ein Verfahren zur Herstellung eines Marknagelspacers.

Zur Behandlung von Knochenfrakturen, beispielsweise in der Unfallchirurgie, werden Marknägel (intramedulläre Nägel) verwendet. Dies erfolgt insbesondere bei Frakturen von Röhrenknochen wie den Oberschenkelknochen (Femur), Schienbeinknochen (Tibia) oder den Oberarmknochen (Humerus). Marknägel werden typischerweise längs in den Hohlraum des Knochens eingetrieben und ermöglichen dort die Neubildung von Knochengewebe. Nach derartiger Versorgung kann es zu Infektionen des umliegenden Knochen- und/oder Weichgewebes kommen, beispielsweise mit Bakterien wie Staphyloccus aureus oder Staphylococus epdidermidis. Dies ist insbesondere bei offenen Frakturen der Fall und macht eine chirurgische Sanierung notwendig, bei der der Marknagel entnommen wird und infiziertes Gewebe abgetragen wird (Debridement). Zudem werden eine systemische Antibiose und/oder lokale Antiinfektiva eingesetzt, um die Infektion gezielt zu bekämpfen.

Es ist bekannt, Marknägel mit einer Antibiotika enthaltenden Beschichtung aus Polymethylmethacrylat-Knochenzement zu versehen (N. Walter et al.: "Individual and commercially available antimicrobial coatings for intramedullary nails for the treatment of infected long bone non-unions - a systematic review"; Injury 2022; DOI: 10.1016/j.injury.2022.05.008). Antibiotika aus dem Knochenzement werden durch wässrige Körperflüssigkeiten, wie Wundsekret und Blut, aus der Beschichtung herausgelöst und führen zu lokal hohen Antibiotika-Konzentration. In Kombination mit der systemischen Antibiose können die nach dem Debridement möglicherweise noch verbliebenen Keime reduzieren. Problematisch ist es jedoch, eine gleichmäßige Schichtdicke der Beschichtung zu realisieren. Zu dick beschichtete Marknägel lassen sich nicht oder nur schwer in die zuvor debridierten Röhrenknochen implantieren.

Es ist wünschenswert, wenn nach der chirurgischen Sanierung und vor dem erneuten Einsetzen eines Marknagels eine vorübergehende mechanische Stabilisierung des Markraums möglich ist und auch hier lokal Antiinfektiva abgegeben werden könnte, um im debridierten Gewebe verbliebene mikrobielle Keime lokal zu unterdrücken. Hierzu ist es möglich, intraoperativ und manuell Stäbe aus Polymethylmethacrylat-Knochenzement zu formen, um die zuvor explantierten Marknägel ansatzweise nachzubilden. Auch hier kann ein Antibiotikum zur lokalen Freisetzung vorgesehen werden. Allerdings ist die Passung manuell geformter Stäbe grundsätzlich problematisch. Die manuelle Formgebung ist insbesondere im Falle gekrümmter und/oder größerer Stäbe nicht geeignet, einen Marknagel ausreichend abzubilden. Aus diesem Grund sind die Ergebnisse häufig wenig befriedigend.

Die oben genannten Merkmale können beliebig mit den unterschiedlichen Aspekten der Erfindung kombiniert werden.

Es ist die Aufgabe der Erfindung, verbessert Marknagelspacer herzustellen.

Die Aufgabe wird gelöst durch die Vorrichtung gemäß Anspruch 1 sowie durch die Verwendung und das Verfahren gemäß den nebengeordneten Ansprüchen. Vorteilhafte Ausgestaltungen sind in den Unteransprüchen angegeben.

Zur Lösung der Aufgabe dient eine Vorrichtung zur Herstellung eines Marknagelspacers. Die Vorrichtung umfasst ein erstes Rohr, ein zweites Rohr und ein flexibles Verbindungsstück zum strömungstechnischen Verbinden des ersten Rohrs mit dem zweiten Rohr. Das erste Rohr und das zweite Rohr sind in unterschiedlichen Winkeln zueinander positionierbar.

Die Vorrichtung erlaubt es, auf einfache und definierte Weise Marknagelspacer (im Folgenden auch kurz als Spacer bezeichnet) herzustellen. Ein Marknagelspacer kann anstelle des entfernten Marknagels eingesetzt werden, um nach der chirurgischen Sanierung und vor dem erneuten Einsetzen eines Marknagels eine vorübergehende mechanische Stabilisierung des Markraums zu ermöglichen.

Ein Marknagelspacer kann aus einer Gussmasse hergestellt werden, beispielsweise Knochenzement. Ein Marknagelspacer kann einen oder mehrere Wirkstoffe wie z. B. Antiinfektiva, etwa Antibiotika, enthalten, um diese lokal abzugeben. Wirkstoffe können der Gussmasse beigegeben werden, um in den Marknagelspacer eingebaut zu werden. Die Vorrichtung dient als Gussform. Die Gussmasse kann in die Vorrichtung eingefüllt werden und darin aushärten, um einen Marknagelspacer zu bilden. Anschließend kann der ausgehärtete Marknagelspacer aus der Vorrichtung entfernt werden. Dabei können insbesondere die einzelnen Rohr sowie das Verbindungsstück einzeln vom Marknagelspacer entfernt werden. Dabei kann ein zerstörungsfreies Abziehen möglich sein, beispielsweise bei einem oder beiden Rohren und/oder dem Verbindungsstück, und/oder es kann ein Abtrennen unter Zerstörung der jeweiligen Gussform, beispielsweise des Verbindungsstücks, erfolgen.

Marknägel können je nach vorgesehenem Implantationsort als gerade oder gekrümmte Hohlköper ausgebildet sein. Die Positionierung des ersten Rohrs und des zweiten Rohrs in unterschiedlichen Winkeln zueinander ermöglicht, die ggf. vorhandene Krümmung des explantierten Marknagels nachzubilden. Es können je nach Anforderung gerade oder beliebig gekrümmte Marknagelspacer hergestellt werden. So kann der entfernte Marknagel auch intraoperativ nachgebildet werden, um möglichst gut an die vorgegebene Position im Markraum zu passen. Die Intraoperative Herstellung ermöglicht, exakt auf die vorliegenden Keime abgestimmte Wirkstoffe, etwa Antiinfektiva wie z.B. Antibiotika, in den Spacer einzubringen.

Ein Rohr ist ein länglicher Hohlkörper, dessen Länge um einen Faktor von wenigstens 3 größer ist als sein äußerer Querschnitt oder Durchmesser. Ein Rohr definiert in seinem Inneren einen länglichen Hohlraum. Ein innerer und/oder äußerer Querschnitt des Rohrs kann kreisförmig sein, grundsätzlich sind aber auch abweichende Querschnitte wie ovale, quadratische, rechteckige oder andere insbesondere regelmäßige polygonale Querschnitte möglich. Üblicherweise entsprechen der innere und der äußere Querschnitt einander. Die Wandstärke kann in axialer Richtung und/oder in Umfangsrichtung konstant sein.

Ein Hohlraum im ersten Rohr und/oder im zweiten Rohr kann konisch geformt sein. Im Falle eines Kreisquerschnitts kann der innere Durchmesser entlang der axialen Richtung kontinuierlich steigen. Konische Rohre sind leichter entformbar. Mit anderen Worten ist das Lösen des hergestellten Teils des Marknagelspacers aus dem betreffenden Rohr einfacher möglich. In einigen Ausführungsformen enthält das Rohr im Inneren keine Sprünge. Insbesondere weist ein konisches Rohr einen Winkel von höchstens 7° auf, wobei der Winkel zwischen der Längsachse des Rohrs und der Innenwand des Rohrs gemessen wird.

Das erste Rohr ist insbesondere ein distales Rohr, also ein Rohr, welches zur Herstellung eines distalen Abschnitts eines Marknagelspacers eingerichtet ist. Das zweite Rohr ist insbesondere ein proximales Rohr, also ein Rohr, welches zur Herstellung eines proximalen Abschnitts eines Marknagelspacers eingerichtet ist. Das erste Rohr ist insbesondere länger als das zweite Rohr.

Das Verbindungsstück dient dem Verbinden des ersten Rohrs mit dem zweiten Rohr und/oder dem Herstellen eines Verbindungsteils zwischen dem ersten Abschnitt und dem zweiten Abschnitt des Marknagelspacers. Eine strömungstechnische Verbindung ist eine Verbindung, bei welcher der Hohlraum des ersten Rohrs mit dem Hohlraum des zweiten Rohrs verbunden wird. Insbesondere erfolgt die Verbindung über zumindest nahezu den gesamten Querschnitt des ersten Rohrs und/oder des zweiten Rohrs. Das Verbindungsstück ist insbesondere rohrförmig ausgestaltet und kann hohlzylinderförmig und/oder konisch (in Form eines hohlen Kegelstumpfs) geformt sein. Im Falle eines konischen Verbindungsstücks können Marknagelspacer hergestellt werden, deren Abschnitte unterschiedliche Durchmesser aufweisen. Dies kann in Abhängigkeit der Anatomie des Patienten wünschenswert sein.

Insbesondere wird ein Ende des ersten Rohrs mit einer Seite des Verbindungsstücks und ein Ende des zweiten Rohrs mit der anderen Seite des Verbindungsstücks verbunden. Insbesondere erfolgt die Verbindung derart, dass im verbundenen Zustand ein verbundenes Rohr mit zwei Enden und einem durchgehenden Hohlraum vorliegt.

Das Verbindungsstück kann mit dem ersten Rohr und/oder dem zweiten Rohr verbunden oder verbindbar sein. Wenn das Verbindungsstück mit dem ersten Rohr und/oder dem zweiten Rohr verbindbar ist, können die jeweiligen Komponenten separat vorliegen und vom Anwender verbunden werden. Die Vorrichtung kann also auch im demontierten Zustand als System vorliegen. Unabhängig vom verbundenen oder separaten Zustand bei der Auslieferung ist die Verbindung zwischen dem Verbindungsstück und einem jeweiligen Rohr bevorzugt lösbar, insbesondere manuell lösbar, um nach dem Aushärten der Gussmasse ein Entformen zu vereinfachen.

Das Verbindungsstück ist derart flexibel, dass ein Winkel von 40° zwischen dem ersten Rohr und dem zweiten Rohr und damit zwischen den gegenüberliegenden Seiten des Verbindungsstücks eingestellt werden kann. Der Winkel wird gemessen zwischen jeweiligen Längsachsen.

Insbesondere kann die Positionierung in einem bestimmten Winkel durch relative Rotation des ersten Rohrs in Bezug zum zweiten Rohr erfolgen.

Insbesondere ist das erste Rohr und/oder das zweite Rohr gerade. Es ist jedoch ebenso möglich, dass das erste Rohr und/oder das zweite Rohr gekrümmt ist. Beispielsweise kann nahe dem Verbindungsstück eine Krümmung vorliegen und/oder in einem weiter vom Verbindungsstück entfernten Bereich kann ein gerader Rohrabschnitt vorliegen.

Es wird in erster Linie die Anpassung des herzustellenden Marknagelspacers an den entfernten Marknagel beschrieben. Es ist jedoch selbstverständlich ebenso möglich, einen Spacer mit einer anderen Form herzustellen. Insbesondere ist die Vorrichtung zur einmaligen Verwendung vorgesehen.

In einer Ausgestaltung weist die Vorrichtung ferner eine Fixierungseinrichtung zur Fixierung eines Winkels zwischen dem ersten Rohr und dem zweiten Rohr auf. Dies erlaubt, den eingestellten Winkel während des Gießens zu fixieren. Insbesondere kann jeder eingestellte Winkel zumindest näherungsweise fixiert werden. Ein unplanmäßiges Verändern des Winkels kann so effektiv verhindert werden. Die Handhabung während des Gießens wird erleichtert und es können besonders exakte Nachbildungen des explantierten Marknagels hergestellt werden.

Die Fixierungseinrichtung kann beispielsweise dazu eingerichtet sein, das erste Rohr, das zweite Rohr und/oder das Verbindungsstück zu halten und auf diese Weise eine Änderung des Winkels zwischen den Rohren zu verhindern.

Die Fixiereinrichtung kann beispielsweise eine gelochte Platte und eine oder mehrere Stifte umfassen. Die Rohre bzw. das Verbindungsstück können in den gewünschten Winkel bewegt werden und anschließend auf die Platte gelegt werden. Durch Einstecken des Stifts oder der Stifte in die vorgesehenen Löcher kann der Winkel anschließend fixiert werden, indem ein relatives Drehen der Rohre gegeneinander gesperrt wird. Die Löcher können beispielsweise auf einem oder mehreren Kreisbögen angeordnet sein. Abstände zwischen den Löchern können so gewählt sein, dass unterschiedliche Winkel beispielsweise in 5°-Schritten einstellbar sind, beispielsweise zwischen 0° und 40°.Auf der Platte können zudem ein oder mehrere Anlageelemente vorhanden sein, die als feste oder bewegliche Stifte ausgeführt sein können, um ein Rohr, beide Rohre und/oder das Verbindungsstück an einer Seite zu fixieren.

In einer Ausgestaltung weist die Vorrichtung ein Gehäuseunterteil auf, welches einen ersten Gehäuseunterteilabschnitt und einen zweiten Gehäuseunterteilabschnitt umfasst. Insbesondere ist eine relative Drehung der zwei Gehäuseunterteilabschnitte um eine Drehachse möglich. Insbesondere ist der erste Gehäuseunterteilabschnitt zum Halten des ersten Rohrs eingerichtet ist und/oder der zweite Gehäuseunterteilabschnitt ist zum Halten des zweiten Rohrs eingerichtet. Es kann eine Sperreinrichtung vorhanden sein, um eine Drehung der zwei Gehäuseunterteilabschnitte selektiv zu sperren.

Jeder Gehäuseunterteilabschnitt hält ein Rohr. Die beiden Gehäuseunterteilabschnitte können zum Einstellen eines gewünschten Winkels zwischen den Rohren relativ zueinander um die Drehachse rotiert werden. Die Sperreinrichtung dient als Fixierungseinrichtung. Der Winkel zwischen dem ersten Rohr und dem zweiten Rohr wird durch Sperren einer Drehung zwischen den beiden Gehäuseunterteilabschnitten erreicht.

Halten meint ein Blockieren einer Bewegung in zumindest einer Bewegungsrichtung. Insbesondere blockiert die Halteeinrichtung ein axiales Herausziehen des ersten und/oder zweiten Rohrs. Zudem kann die Halteeinrichtung ein senkrecht zur Achse gerichtetes Bewegen des jeweiligen Rohrs und/oder eine Rotation um eine Längsachse und/oder eine senkrecht zur Längsachse ausgerichteten Achse blockieren.

Insbesondere ist der erste Gehäuseunterteilabschnitt zudem zum Halten einer Seite des Verbindungsstücks eingerichtet und/oder der zweiten zweite Gehäuseunterteilabschnitt ist zudem zum Halten einer anderen Seite des Verbindungsstücks eingerichtet. Insbesondere umfasst der erste Gehäuseunterteilabschnitt eine Aufnahme für das erste Rohr und die dem ersten Rohr zugewandte Seite des Verbindungsstücks. Insbesondere umfasst der zweite Gehäuseunterteilabschnitt eine Aufnahme für das zweite Rohr und die dem zweiten Rohr zugewandte Seite des Verbindungsstücks.

Jeder Gehäuseunterteilabschnitt kann im seinem zentralen Bereich eine Scheibe umfassen, wobei die beiden Scheiben flach aufeinander liegen können und gegeneinander drehbar sein können.

Das Gehäuseunterteil kann so ausgebildet sein, dass das erste Rohr, das zweite Rohr das Verbindungsstück und/oder eine beliebige Kombination dieser Komponenten in einer Halteposition in das Gehäuseunterteil eingeführt, eingelegt oder eingesteckt werden können. Das Gehäuseunterteil kann entsprechende Aufnahmen für das erste Rohr, das zweite Rohr und das Verbindungsstück aufweisen, in denen die jeweiligen Komponenten positioniert werden können. Eine Aufnahme kann in einem einfachen Fall gebildet sein durch eine Oberfläche mit zwei oder mehr Stiften oder Vorsprüngen, zwischen denen das jeweilige Rohr oder Verbindungsstück auf die Oberfläche gelegt werden kann. eine Aufnahme kann auch an die äußere Form des Rohrs oder Verbindungsstücks angepasst sein.

Die Drehachse ist insbesondere eine virtuelle Drehachse. Mit anderen Worten ist nicht unbedingt eine körperliche Drehachse notwendig. Die Drehachse verläuft insbesondere durch das Verbindungsstück hindurch.

Das Gehäuseunterteil kann liegt bei bestimmungsgemäßem Gebrauch unten liegen und wird deshalb als Gehäuseunterteil bezeichnet. Dies ist allerdings nicht unbedingt notwendig; die Vorrichtung kann in einer beliebigen Ausrichtung bestimmungsgemäß verwendet werden.

Die Sperreinrichtung kann beispielsweise einen ersten Gehäuseunterteilabschnitt und einen zweiten Gehäuseunterteilabschnitt aneinander fixieren, beispielsweise durch Kraftschluss und/oder Formschluss. Die Sperreinrichtung kann beispielsweise einen Stift umfassen, der in entsprechende Öffnungen eingesteckt wird. Beispielsweise können im ersten Gehäuseunterteilabschnitt und im zweiten Gehäuseunterteilabschnitt eine oder mehrere Öffnungen vorhanden sein, die durch relative Drehung der Gehäuseunterteilabschnitte aneinander ausgerichtet werden können. Anschließend kann der Stift durch eine Öffnung in jedem Gehäuseunterteilabschnitt gesteckt werden, um eine relative Drehung zu sperren.

Alternativ oder ergänzend kann die Sperreinrichtung eine insbesondere manuell zu drehende Schraube vorhanden sein, die im eingeschraubten Zustand die zwei Gehäuseunterteilabschnitte aneinander fixiert. In Ausführungsform kann die Schraube in ein Gewinde eines außen befindlichen Gehäuseunterteilabschnitts eingeschraubt werden und auf einen innen befindlichen Gehäuseunterteilabschnitt drücken. So kann eine kraftschlüssige Fixierung erfolgen. Ist zudem eine Aussparung im innen befindlichen Gehäuseunterteilabschnitt vorhanden, kann zwischen der Schraube und der Aussparung eine formschlüssige Verbindung hergestellt werden.

In einer Ausgestaltung weist die Vorrichtung ein Gehäuseoberteil auf, welches einen ersten Gehäuseoberteilabschnitt und einen zweiten Gehäuseoberteilabschnitt umfasst. Insbesondere ist eine relative Drehung der zwei Gehäuseoberteilabschnitte um die Drehachse möglich. Insbesondere ist das Gehäuseoberteil mit dem Gehäuseunterteil verbunden oder verbindbar, um gemeinsam ein Gehäuse zu bilden. Es kann eine Sperreinrichtung vorhanden sein, um eine Drehung der zwei Gehäuseoberteilabschnitte selektiv zu sperren.

Der erste Gehäuseoberteilabschnitt kann zum Halten des ersten Rohrs beitragen und/oder der zweite Gehäuseoberteilabschnitt kann zum Halten des zweiten Rohrs beitragen der erste und der zweite Gehäuseoberteilabschnitt kann zum Halten des Verbindungsstücks beitragen. Beispielsweise können entsprechende Aussparungen vorhanden sein, in denen das jeweilige Rohr bzw. das Verbindungsstück liegen kann.

Das Gehäuseoberteil kann so ausgebildet sein, dass es mit dem Gehäuseunterteil ein zumindest teilweise geschlossenes, insbesondere kompaktes Gehäuse bildet. Das Gehäuseunterteil und das Gehäuseoberteil können dazu miteinander verbunden sein oder werden, z. B. durch Zusammenstecken. Im verbundenen Zustand kann eine Verdrehung des Gehäuseunterteils und des Gehäuseoberteils gegeneinander blockiert sein. Insbesondere kann das Gehäuseoberteil manuell und/oder zerstörungsfrei von dem Gehäuseunterteil abgenommen werden.

Das Gehäuse weist dann typischerweise eine erste Öffnung für das erste Rohr und eine zweite Öffnung für das zweite Rohr auf. Insbesondere umgibt das Gehäuse das Verbindungsstück und/oder überragt das Verbindungsstück in allen Richtungen. Es kann dann eine relative Drehung des ersten Gehäuseunterteilabschnitts mit dem ersten Gehäuseoberteilabschnitt gegenüber dem zweiten Gehäuseunterteilabschnitt mit dem zweiten Gehäuseoberteilabschnitt erfolgen. Damit erfolgt dann auch eine relative Drehung des ersten Rohres in Bezug zu dem zweiten Rohr.

Jeder Gehäuseoberteilabschnitt kann im seinem zentralen Bereich eine Scheibe umfassen, wobei die beiden Scheiben flach aufeinander liegen können und gegeneinander drehbar sein können.

Insbesondere umfassen das Gehäuseunterteil und das Gehäuseoberteil korrespondierende Formelemente, um eine Verbindung des ersten Gehäuseoberteilabschnitts mit dem ersten Gehäuseunterteilabschnitt und/oder eine Verbindung des zweiten Gehäuseoberteilabschnitts mit dem zweiten Gehäuseunterteilabschnitt zu ermöglichen. Korrespondierende Formelemente können beispielsweise einerseits Vorsprünge, z. B. Stifte, und andererseits Aussparungen, z. B. Sacklöcher sein. Formelemente können so eingerichtet sein, dass ein Zusammenstecken des Gehäuseunterteils und des Gehäuseoberteils möglich ist. Ein Zusammenstecken erfolgt insbesondere parallel zur Drehachse.

In einer Ausgestaltung weist das erste Rohr und/oder das zweite Rohr ein nach außen ragendes Formschlusselement auf. Insbesondere ist das Formschlusselement derart gehalten, dass ein axiales Herausziehen des jeweiligen Rohrs blockiert ist. Insbesondere umfasst die Vorrichtung ein zweites Element, welches das Formschlusselement blockiert.

Das Formschlusselement wirkt insbesondere mit einem Gehäuse oder einem Teil davon zusammen. Beispielsweise kann ein Gehäuseunterteil und/oder ein Gehäuseoberteil ein mit dem Formschlusselement zusammenwirkendes Element aufweisen, welches das Formschlusselement kontaktiert und so ein Herausziehen verhindert. Beispielsweise kann eine Aussparung zur Aufnahme des Formschlusselements vorhanden sein. Insbesondere ragt das Formschlusselement radial nach außen.

Das Halten des Rohrs mit dem Formschlusselement ist insbesondere lösbar. Das bedeutet, dass z. B. nach dem Gießen ein Herausnehmen der Rohre und des Verbindungsstücks einfach möglich ist, beispielsweise nach dem Abnehmen des Gehäuseoberteils vom Gehäuseunterteil.

Das Formschlusselement kann umlaufend um das erste Rohr und/oder das zweite Rohr angeordnet sein. Das Formschlusselement kann beispielsweise als umlaufende Wulst, als umlaufender Steg oder als insbesondere senkrecht zur Längsachse ausgerichtete umlaufende Scheibe ausgestaltet sein. Das Formschlusselement kann eine senkrecht zur Längsachse ausgerichtete Fläche aufweisen, um das Herausziehen zu blockieren.

Insbesondere weist das Verbindungsstück ebenfalls mindestens ein nach außen ragendes Formschlusselement auf, welches derart gehalten ist, dass ein axiales Herausziehen des jeweiligen Rohrs blockiert. Auch dieses Formschlusselement kann von einem Gehäuse oder einem Teil davon gehalten sein. Das Formschlusselement des Verbindungsstücks kann so ausgebildet sein wie das Formschlusselement des ersten und/oder zweiten Rohrs. In einem Ausführungsbeispiel weist das Verbindungsstück zwei Formschlusselemente auf, wobei ein erstes Formschlusselement von dem ersten Gehäuseoberteilabschnitt und/oder dem ersten Gehäuseunterteilabschnitt gehalten wird und ein zweites Formschlusselement von dem zweiten Gehäuseoberteilabschnitt und/oder dem zweiten Gehäuseunterteilabschnitt gehalten wird. Dies verhindert ein Lösen der Verbindung zwischen dem Verbindungsstück und jedem der Rohre auch bei einem erhöhten Einpressdruck der Gussmasse.

In einer Ausgestaltung ist eine Länge des ersten Rohrs und/oder eine Länge des zweiten Rohrs einstellbar. Auf diese Weise kann der Marknagelspacer auch hinsichtlich der Erstreckung des jeweiligen Abschnitts bzw. der Abschnitte an den entfernten Marknagel und damit an die Anatomie des Patienten angepasst werden. So kann eine besonders genaue Nachbildung des entfernten Marknagels erreicht werden. Es sind grundsätzlich unterschiedlichste Möglichkeiten geeignet, um die Länge des jeweiligen Rohrs einzustellen.

In einer Ausgestaltung ist das erste Rohr und/oder das zweite Rohr für eine Längeneinstellung teleskopartig ausgebildet. Die Länge des jeweiligen Rohrs kann auf diese Weise teleskopartig eingestellt werden. Insbesondere umfasst das betreffende Rohr ein äußeres Rohr mit einem größeren ersten Querschnitt oder Durchmesser und ein separates inneres Rohr mit einem kleineren zweiten Querschnitt oder Durchmesser, die entlang einer gemeinsamen Längsachse ausgerichtet und insbesondere miteinander verbunden sind. Das betreffende erste bzw. zweite Rohr ist somit ein zusammengesetztes oder mehrteiliges Rohr. Es kann eine Relativbewegung zwischen dem inneren Rohr und dem äußeren Rohr entlang der Längsachse erfolgen, um die Gesamtlänge einzustellen. Mit anderen Worten kann das innere Rohr in dem äußeren Rohr bewegt werden. Das äußere Rohr ist insbesondere mit dem Verbindungsstück verbunden und/oder das innere Rohr ist vom Verbindungsstück abgewandt. So können beide Rohrteile nach dem Aushärten zusammen oder einzeln nach außen abgezogen werden. Etwaige Verschlusselemente oder andere Komponenten können am vom Verbindungsstück abgewandten Rohr angeordnet sein.

Insbesondere umfasst die Vorrichtung ferner eine Befestigungseinrichtung zur Befestigung des innere Rohr an dem äußeren Rohr in einer gewünschten Position. So kann ein unplanmäßiges Verändern der Länge verhindert werden. Die Befestigungseinrichtung kann beispielsweise eine Schraube umfassen, die in ein Gewinde am äußeren Rohr eingeschraubt werden kann und mit einem vorderen Kontaktbereich das innere Rohr kontaktiert. Die Schraube kann senkrecht zur Längsachse des jeweiligen Rohrs ausgerichtet sein. So kann eine kraftschlüssige Verbindung hergestellt werden. Das innere Rohr kann an seiner Außenwand Formelemente aufweisen, die mit dem Kontaktbereich in Eingriff kommen können. So kann eine formschlüssige Verbindung gegen ein unplanmäßiges Herausziehen hergestellt werden.

In einer Ausgestaltung kann das erste Rohr und/oder das zweite Rohr zum Einstellen der Länge gekürzt werden. Insbesondere weist die Vorrichtung ein Abschlusselement zum Anordnen an der gekürzten Seite auf.

Das Kürzen erfolgt insbesondere mittels eines Trennverfahrens, wie beispielsweise Schneiden oder Sägen. Es wird also ein nicht benötigter Teil des Rohrs abgetrennt. Das Abschlusselement ist insbesondere so eingerichtet, dass eine durch das Trennen hergestellte stirnseitige Schnittfläche des betreffenden Rohrs verdeckt wird. insbesondere erstreckt sich das Abschlusselement in axialer Richtung des betreffenden Rohrs über mindestens 2 cm, so dass ein exakt rechtwinkliges Trennen nicht erforderlich ist und/oder etwaige Ungenauigkeiten verdeckt sind. So wird die Anwendung durch den Benutzer erleichtert.

Der Benutzer kann dann beispielsweise intraoperativ ein Rohr oder beide Rohre an der bzw. den benötigten Längenpositionen abschneiden und die Schnittflächen durch jeweilige Abschlusselemente abdecken. Die Abschlusselemente können zum kraftschlüssigen, formschlüssigen und/oder stoffschlüssigen Verbinden mit dem betreffenden Rohr eingerichtet sein. Das Abschlusselement kann zum Aufstecken und/oder Aufschrauben auf das gekürzte Rohr dienen.

Das Abschlusselement kann als Adapter zur Anordnung eines oder mehrerer weiterer Teile dienen. Das Abschlusselement kann an der dem Rohr abgewandten Seite einen Befestigungsbereich aufweisen, der beispielsweise als Gewinde oder als Teil einer Bajonettverbindung ausgebildet ist. Beispielsweise kann ein Verschlusselement am Befestigungsbereich befestigt werden, um das Rohr zu verschließen.

In einer Ausgestaltung weist das erste Rohr und/oder das zweite Rohr an seiner Außenseite Formelemente zur Herstellung einer Rastverbindung auf. Die Formelemente sind insbesondere in Abständen entlang der Längsachse des Rohrs angeordnet. Insbesondere weist das Abschlusselement ein oder mehrere Rastelemente auf, die mit den jeweiligen Formelementen verrastet werden können. Ein solches Abschlusselement kann auch als Rastmuffe bezeichnet werden.

Jedes Rastelement kann mit einem oder mehreren Formelementen verrastet werden, die insbesondere an derselben axialen Position angeordnet sind. So kann nach dem Kürzen des Rohrs das Abschlusselement auf das gekürzte Rohr aufgeschoben und mit diesem verrastet werden und ein unplanmäßiges Abziehen des Abschlusselements insbesondere durch Formschluss verhindert werden.

Die Rastelemente können hakenförmig sein und/oder eine in Bezug zur Längsachse des betreffenden Rohrs beispielsweise senkrechte oder hinterschnittene Rastfläche aufweisen. Die Formelemente können hakenförmig sein und/oder eine beispielsweise senkrechte oder hinterschnittene Rastfläche aufweisen. Eine hinterschnittene Rastfläche meint eine Rastfläche, die so geneigt ist, dass bei Auftreten einer axialer Kraft, die die Rohr auseinanderzuziehen versucht, eine Selbsthemmung eintritt. Eine hinterschnittene Rastfläche weist typischerweise einen Winkel größer als 90° zur Längsachse auf.

Die Rastelemente können als umlaufende Nuten ausgebildet sein. In diesem Fall kann die Rastverbindung unabhängig von der relativen Position zwischen dem betreffenden Rohr und dem Abschlusselement hergestellt werden. Die umlaufenden Nuten können sägezahnförmig sein, beispielsweise mit einer senkrechten und einer schrägen Fläche.

In einer Ausgestaltung umfasst das Abschlusselement ein Klemmteil mit einem Klemmbereich zum Einklemmen des gekürzten Rohrs, wobei das Klemmteil ein erstes Gewinde, z. B. ein Außengewinde aufweist. Insbesondere kann das Rohr in den Klemmbereich eingesteckt werden. In einer Ausgestaltung umfasst das Abschlusselement eine auf das Rohr aufsteckbare Überwurfmutter mit einem zweiten Gewinde, z. B. einem Innengewinde. Insbesondere kann das gekürzte Rohr durch Aufschrauben der Überwurfmutter auf das Klemmteil bzw. durch Verbinden des zweiten Gewindes und des ersten Gewindes im Klemmbereich festgeklemmt werden.

Dies ermöglicht auf einfache Weise eine kraftschlüssige Verbindung zwischen Abschlusselement und Rohr. Es können beliebige Längen eingestellt werden.

Der Klemmbereich kann in Umfangsrichtung verteilte, ggf. zahnartige flexible Klemmelemente umfassen, die flexibel radial nach außen oder innen bewegt werden können, wenn das Rohr in den Klemmbereich eingesteckt wird. Die Klemmelemente können durch axiale Schlitze hergestellt worden sein. Das Rohr befindet sich dann in seinem Endabschnitt zwischen den Klemmteilen und einem gegenüberliegenden Teil des Abschlusselements. Durch Aufschrauben der Überwurfmutter können die Klemmelemente und das gegenüberliegende Teil aneinander gepresst werden, um das Rohr im Klemmbereich festzuklemmen.

In einer Ausgestaltung umfasst die Vorrichtung ein erstes Endstück zur Anordnung an einem dem Verbindungsstück abgewandten Ende des ersten Rohrs oder des zweiten Rohrs. Insbesondere ist an einer dem ersten Rohr bzw. dem zweiten Rohr abgewandten Seite des ersten Endstücks ein Verbindungsbereich zum Verbinden mit einer Gussmassequelle angeordnet.

In einer Ausgestaltung umfasst die Vorrichtung ein zweites Endstück zur Anordnung an einem dem Verbindungsstück abgewandten Ende des ersten Rohrs oder des zweiten Rohrs. Insbesondere verschließt das zweite Endstück das Rohr teilweise. Insbesondere ist im zweiten Endstück eine Entlüftungsöffnung angeordnet, die das Rohr mit der Umgebung verbindet.

Das erste Endstück dient insbesondere als Adapter zum Verbinden mit einer Gussmassequelle. Das erste Endstück ist in axialer Richtung typischerweise durchlässig. Durch das erste Endstück hindurch kann die Gussmasse aus der Gussmassequelle in die Vorrichtung gegeben werden, um einen Spacer herzustellen. Die Gussmassequelle kann beispielsweise eine Kartusche sein, in der Knochenzement hergestellt worden ist. Eine Gussmassequelle kann direkt oder mit einem zwischengeschalteten Rohr oder Schlauch verbunden werden. Insbesondere wird die Gussmasse aus der Gussmassequelle in die Vorrichtung gedrückt. Der Verbindungsbereich kann beispielsweise als Innengewinde oder Außengewinde ausgebildet sein. Ein Schlauch oder Rohr der Kartusche kann auch einfach in eine entsprechend Öffnung des Endstücks eingesteckt werden.

Das erste Endstück und/oder das zweite Endstück kann nach Beenden des Einfüllvorgangs insbesondere verschlossen werden. Beispielsweise kann an dem Verbindungsbereich oder an einem anderen Bereich des ersten Endstücks eine Verschlusskappe oder ein Stopfen angeordnet werden. So können überstehender Anguss bzw. austretende Gussmasse verhindert und ein sauberer Abschluss des Marknagelspacers erreicht werden. Das zweite Endstück kann zu diesem Zweck ebenfalls einen Verbindungsbereich aufweisen.

In einer Ausführungsform umfasst die Vorrichtung ein Verschlusselement zum Verschließen des ersten Rohrs, des zweiten Rohrs, des ersten Endstücks, des zweiten Endstücks und/oder eines Abschlusselements. So kann das jeweilige Rohr an der dem Verbindungsstück abgewandten Seite verschlossen werden. Das Verschlusselement kann etwa eine Verschlussschraube mit einem Außengewinde oder eine Schraubkappe mit Innengewinde sein.

Ein Verschlusselement kann auch als Stopfen, etwa aus Kunststoff oder Elastomer, ausgestaltet sein. Das den Stopfen aufnehmende Teil und/oder der Stopfen kann sich konisch verjüngen, um ein Einpressen zu erleichtern.

Das zweite Endstück dient insbesondere als Verschluss und/oder Entlüftung. Wird Gussmasse in die Vorrichtung gegeben, muss die verdrängte Luft aus der Vorrichtung entweichen. Hierzu ist die Entlüftungsöffnung vorgesehen. Die Entlüftungsöffnung hat insbesondere einen Querschnitt oder Durchmesser von einem oder wenigen Millimetern.

Das erste Endstück und/oder das zweite Endstück kann direkt oder indirekt an dem jeweiligen Rohr angeordnet sein oder werden. Bei einer indirekten Anordnung können ein oder mehrere Zwischenstücke vorhanden sein. Beispielsweise kann ein oben beschriebenes Abschlusselement als Zwischenstück zwischen dem Rohr und dem Endstück angeordnet sein, auch wenn ein Kürzen des Rohres nicht vorgesehen ist. Alternativ kann das Abschlusselement als erstes oder zweites Endstück fungieren. Das erste Endstück und/oder das zweite Endstück kann einteilig oder mehrteilig ausgeführt sein.

Das erste Endstück und/oder das zweite Endstück kann einen Durchlass für ein Inlay aufweisen. Ein Inlay kann so durch das Endstück hindurch geführt werden, beispielsweise, um auf diese Weise gehalten zu werden. Ein teilweises Hindurchführen ist ebenfalls möglich; hierbei endet das Inlay im Durchlass. Der Durchlass kann im verbundenen Zustand durch das Inlay teilweise oder vollständig verschlossen bzw. abgedichtet sein. Der Durchlass kann hinsichtlich seiner Erstreckung, insbesondere seines Querschnitts, auf das Inlay abgestimmt sein.

Der Durchlass kann mindestens eine Aussparung umfassen, beispielsweise in Form einer Nut, wobei die Aussparung in axialer Richtung durch ein Gewinde eines Verbindungsbereichs der ersten bzw. zweiten Rohrs verläuft. Befindet sich das Inlay im Durchlass, kann eine weitere Komponente mit einem korrespondierenden Gewinde auf das Gewinde auch bei vorhandenem Inlay aufgeschraubt werden, ohne dass Scherkräfte auf das Inlay wirken. Ist ein Stopfen vorgesehen, kann das den Stopfen aufnehmende Teil in ebenfalls axialer Richtung durch den Verbindungsbereich verlaufen. Sind mehrere Aussparungen oder Nuten vorhanden, beispielsweise in Umfangsrichtung verteilt, ist ein Einfädeln erleichtert.

Insbesondere ist die Vorrichtung so eingerichtet, dass ein innerer Querschnitt oder Durchmesser im an das Verbindungsstück angrenzenden Bereich des ersten Rohrs zumindest ungefähr dem inneren Querschnitt des benachbarten Abschnitts des Verbindungsstücks entspricht und/oder dass ein innerer Querschnitt oder Durchmesser im an das Verbindungsstück angrenzenden Bereich des zweiten Rohrs zumindest ungefähr dem inneren Querschnitt des benachbarten Abschnitts des Verbindungsstücks entspricht. So kann ein im Wesentlichen konstanter äußerer Querschnitt bzw. Durchmesser des Marknagelspacers hergestellt werden.

In einer Ausgestaltung weist das Verbindungsstück einen ersten Anschlussbereich zum Aufstecken auf das erste Rohr, einen zweiten Anschlussbereich zum Aufstecken auf das zweite Rohr und einen zwischen den Anschlussbereichen befindlichen mittleren Bereich auf. Insbesondere ist ein innerer Querschnitt des Verbindungsstücks im mittleren Bereich kleiner als im ersten und zweiten Anschlussbereich.

Das Verbindungsstück ist hierbei als Muffe ausgebildet, also als außen am Rohr angeordnetes Verbindungsstück. Das Verbindungsstück kann auf das erste Rohr und/oder das zweite Rohr aufgeschoben werden. Insbesondere kann das Verbindungsstück anschließend vom ersten Rohr und/oder vom zweiten Rohr abgezogen werden.

Das Verbindungsstück kann aus einem flexiblen Material, einem Elastomer und/oder einem Silikon hergestellt sein, zum Beispiel aus einem biokompatiblen gummielastischen Kunststoff wie zum Beispiel Silikonkautschuk oder EPDM-Kautschuk. Das Verbindungsstück ist insbesondere aus einem Material mit einer Shore A-Härte von gleich oder größer 50.

Insbesondere ist das Verbindungsstück so flexibel, dass es im jeweiligen Anschlussbereich zumindest bereichsweise einen kleineren Innendurchmesser aufweist als der Außendurchmesser des entsprechenden Rohrs. Das Verbindungsstück kann dann aufgrund einer radial nach innen gerichteten Rückstellkraft an dem Rohr kraftschlüssig befestigt sein. Auf diese Weise kann ein Austreten von Gussmasse zwischen Verbindungsstück und Rohr verhindert werden, auch bei einem erhöhten Einpressdruck der Gussmasse. Zudem kann ein unplanmäßiges Lösen verhindert werden.

Insbesondere ist das erste Rohr und/oder das zweite Rohr aus einem Material mit einer im Vergleich zum Verbindungsstück größeren Steifigkeit und/oder Festigkeit und/oder aus einem thermoplastischen Kunststoff hergestellt.

In einer Ausführungsform sind das erste Rohr, das zweite Rohr und/oder das Verbindungsstück aus einem transluzenten und/oder transparenten Material hergestellt. So kann der Befüllvorgang visuell kontrolliert werden.

Insbesondere entspricht die Differenz der Innendurchmesser der zweifachen Wandstärke des jeweiligen Rohrs. Auf diese Weise kann auch im Übergangsbereich zwischen dem Verbindungsstück und dem jeweiligen Rohr eine durchgehende Außenfläche des Spacers ohne Sprünge hergestellt werden.

Alternativ dazu kann das Verbindungsstück auch Anschlussbereiche aufweisen, die zum Einstecken in das jeweilige Rohr eingerichtet sind. In diesem Fall kann das jeweilige Rohr in dem Verbindungsbereich einen vergrößerten Innendurchmesser aufweisen, um eine konstante Innenfläche des Zusammenschlusses zu ermöglichen.

In einer Ausgestaltung umfasst die Vorrichtung ein in dem ersten Rohr und/oder dem zweiten Rohr angeordnetes Inlay zum inneren Verstärken des herzustellenden Marknagelspacers.

Das Inlay ist insbesondere aus Metall hergestellt und kann auch als Metallkern bezeichnet werden. Das Inlay dient der mechanischen Verstärkung des Marknagelspacers. Insbesondere ist das Inlay in dem betreffenden Rohr etwa mittig angeordnet, um anschließend etwa mittig im Spacer zu liegen. Das Inlay kann mit geeigneten Haltern in dem betreffenden Rohr gehalten werden. Insbesondere sind in Umfangsrichtung mindestens drei Halter oder Abstandhalter vorhanden, um das Inlay etwa mittig zu halten. Abstandhalter können am Inlay befestigt sein. Das Inlay kann genau mittig angeordnet sein. Es ist jedoch akzeptabel, wenn das Inlay sich in einem zentralen Bereich befindet, der der Hälfte des Innenradius des betreffenden Rohrs entspricht.

Das Inlay kann sich über einen Teil der Länge des ersten und/oder zweiten Rohrs oder über die jeweilige gesamte Länge erstrecken. Es kann zudem ein Inlay bzw. das Inlay im Verbindungsstück angeordnet sein. In einer Ausführungsform ist ein durchgehendes Inlay angeordnet, dass sich vom ersten Rohr über das Verbindungsstück bis in das zweite Rohr erstreckt. Als Inlay kann z. B. ein Kirschner-Draht verwendet werden.

Grundsätzlich ist eine mechanische Stabilisierung des Spacers nicht immer notwendig, weil auf Grund der Schwere der Erkrankung eine Mobilisierung der Patienten in vielen Fällen nicht möglich ist. In diesem Fall werden keine Inlays benötigt. Es kann dennoch vorgesehen sein, standardmäßig für alle Spacer ein Inlay vorzusehen. Dies kann den Aufwand reduzieren, da nur eine Vorrichtung für alle Anwendungen verwendbar ist.

Ein weiterer Aspekt der Erfindung ist ein Kit, umfassend:
- eine erfindungsgemäße Vorrichtung, sowie
- mindestens ein weiteres erstes Rohr, wobei das weitere erste Rohr eine andere Länge, eine andere Form und/oder einen anderen Querschnitt, insbesondere Durchmesser, aufweist als das erste Rohr der Vorrichtung, wobei das weitere erste Rohr anstelle des ersten Rohrs mit dem Verbindungsstück verbunden werden kann, oder
- mindestens ein weiteres zweites Rohr, wobei das weitere zweite Rohr eine andere Länge, eine andere Form und/oder einen anderen Querschnitt, insbesondere Durchmesser, aufweist als das zweite Rohr der Vorrichtung, wobei das weitere zweite Rohr anstelle des zweiten Rohrs mit dem Verbindungsstück verbunden werden kann.

Der Kit enthält also mehrere erste und/oder zweite Rohre, so dass das geeignete Rohr ausgewählt werden kann, das am besten geeignet ist, um den explantierten Marknagel nachzubilden.

Ein weiterer Aspekt der Erfindung ist die Verwendung einer Gussform zur Herstellung eines Marknagelspacers. Insbesondere wird eine erfindungsgemäße Vorrichtung als Gussform verwendet. Alle Merkmale, Vorteile und Ausgestaltungen der eingangs beschriebenen Vorrichtung gelten ebenso für die Verwendung und umgekehrt. Insbesondere wird Polymethylmethacrylat-Knochenzement in die Gussform gegeben.

Ein weiterer Aspekt der Erfindung ist ein Verfahren zur Herstellung eines Marknagelspacers unter Verwendung einer Vorrichtung, wobei die Vorrichtung ein erstes Rohr, ein zweites Rohr und ein flexibles Verbindungsstück zum strömungstechnischen Verbinden des ersten Rohrs mit dem zweiten Rohr umfasst. Das Verfahren umfasst relatives Positionieren des ersten Rohrs und des zweiten Rohr in einem gewünschten Winkel zueinander, sowie Einfüllen einer Gussmasse in ein erstes Rohr, das zweite Rohr und das Verbindungsstück zur Herstellung des Marknagelspacers. Das Einfüllen erfolgt insbesondere so, dass der gesamte Innenraum der Vorrichtung, also beide Rohre sowie das Verbindungsstück, vollständig gefüllt sind. Das Einfüllen kann durch Einpressen erfolgen. Alle Merkmale, Vorteile und Ausgestaltungen der eingangs beschriebenen Vorrichtung gelten ebenso für das Verfahren und umgekehrt. Insbesondere umfasst die Gussmasse Polymethylmethacrylat-Knochenzement und ggf. ein oder mehrere medizinische Wirkstoffe.

Das Verfahren kann ferner eine oder mehrere der folgenden Schritte in beliebigen Kombinationen umfassen:
- Einstellen einer Länge des ersten Rohrs und/oder des zweiten Rohrs, etwa durch Kürzen des betreffenden Rohrs und Anordnen eines Abschlusselements und/oder durch teleskopartige Längeneinstellung, und ggf. Feststellen der eingestellten Länge,
- Fixieren des eingestellten Winkels mittels einer Fixierungseinrichtung,
- Verbinden eines Rohrs mit einer Gussmittelquelle, beispielsweise mittels einem ersten Endstücks,
- Nach dem Einfüllen: Entfernen der Gussmittelquelle und ggf. Verschließen des Rohrs oder der Komponente, an dem die Gussmittelquelle angeordnet war,
- Entfernen von überschüssigem Gussmittel von dem Ende der Vorrichtung, an dem nicht die Gussmittelquelle angeordnet war, und/oder Verschließen der an diesem Ende befindlichen Komponente,
- Aushärten der Gussmasse,
- Entformen des hergestellten Spacers, beispielsweise durch Abziehen des ersten und/oder zweiten Rohrs vom Spacer, ggf. Aufschneiden des Verbindungsstücks und/oder Abziehen des Verbindungsstücks vom Spacer.
- Entnahme des Spacers.

Nachfolgend werden Ausführungsbeispiele der Erfindung auch anhand von Figuren näher erläutert. Merkmale der Ausführungsbeispiele können einzeln oder in einer Mehrzahl mit den beanspruchten Gegenständen kombiniert werden, sofern nichts Gegenteiliges angegeben ist. Die beanspruchten Schutzbereiche sind nicht auf die Ausführungsbeispiele beschränkt.

Es zeigen:
- Figur 1:: eine perspektivische Zeichnung einer Vorrichtung,
- Figur 2:: eine perspektivische Explosionszeichnung einer Vorrichtung,
- Figuren 3 bis 7:: vergrößerte Details,
- Figur 8:: Teile einer Vorrichtung nebst einem hergestellten Spacer, sowie
- Figuren 9 bis 11:: vergrößerte Details.

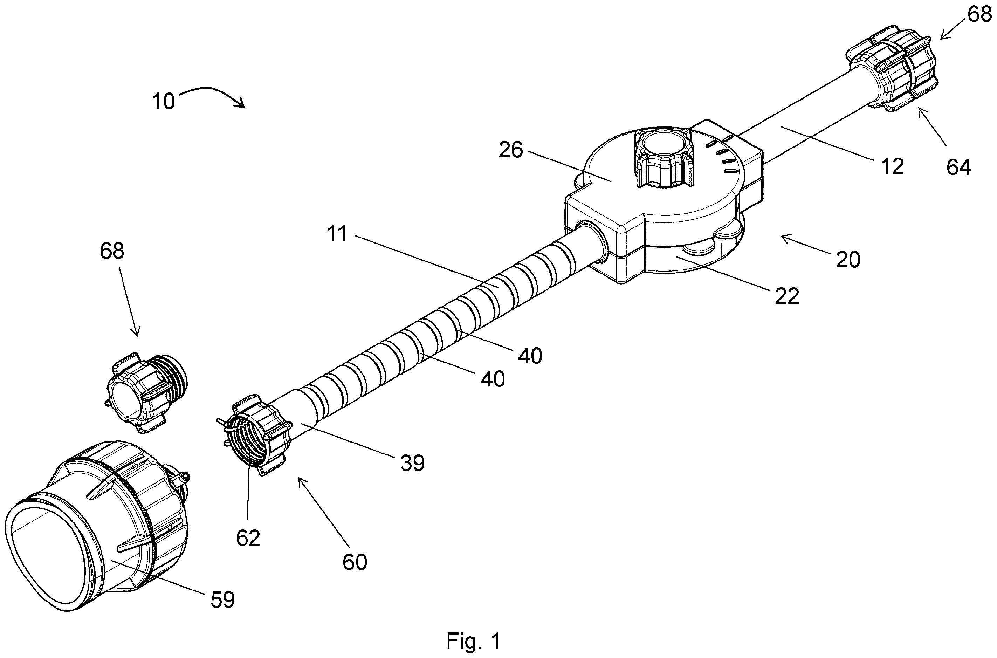

Figur 1 zeigt eine erfindungsgemäße Vorrichtung 10 zur Herstellung eines Marknagelspacers. Die Vorrichtung 10 umfasst ein erstes Rohr 11, ein zweites Rohr 12 sowie ein flexibles Verbindungsstück, welches das erste Rohr 11 mit dem zweiten Rohr 12 strömungstechnisch verbindet. Das Verbindungsstück ist in einem Gehäuse 20 angeordnet, welches ein Gehäuseunterteil 22 und ein Gehäuseoberteil 26 umfasst. Am unten links dargestellten freien Ende des ersten Rohrs 11 ist ein erstes Endstück 60 angeordnet, welches einen Verbindungsbereich 62 mit einem Innengewinde zur mechanischen Verbindung mit einer Gussmassequelle 59 aufweist. Die Gussmassequelle 59 ist hier beispielhaft eine Kartusche mit Knochenzement, die hier teilweise dargestellt ist und Ausgabestutzen mit einem Außengewinde aufweist. Das Außengewinde kann mit dem Innengewinde des Verbindungspreis 62 verbunden werden, um die Gussmasse durch das erste Rohr 11 in das gesamte Innere der Vorrichtung zu pressen. Mit anderen Worten kann die Vorrichtung 10 als Gussform verwendet werden, um aus einer Gussmasse, z. B. Knochenzement, einen Marknagelspacer herzustellen.

Die Vorrichtung 10 umfasst ferner eine Verschlusskappe 68, die nach dem Einfüllen der Gussmasse mit dem Verbindungsbereich 62 verbunden werden kann. Am oben rechts dargestellten freien Ende des zweiten Rohrs 12 ist ein zweites Endstück 64 angeordnet, welches ebenfalls eine Verschlusskappe 68 umfasst.

Das erste Rohr 11 ist typischerweise ein distales Rohr, in dem ein distaler Abschnitt des Marknagelspacers hergestellt wird. Dieses ist üblicherweise länger als das proximale zweite Rohr 12. Typischerweise weist entsprechend dem nachzubildenden Marknagel das zweite Rohr 12 einen Innendurchmesser auf, der mindestens dem Innendurchmesser des ersten Rohrs 11 entspricht.

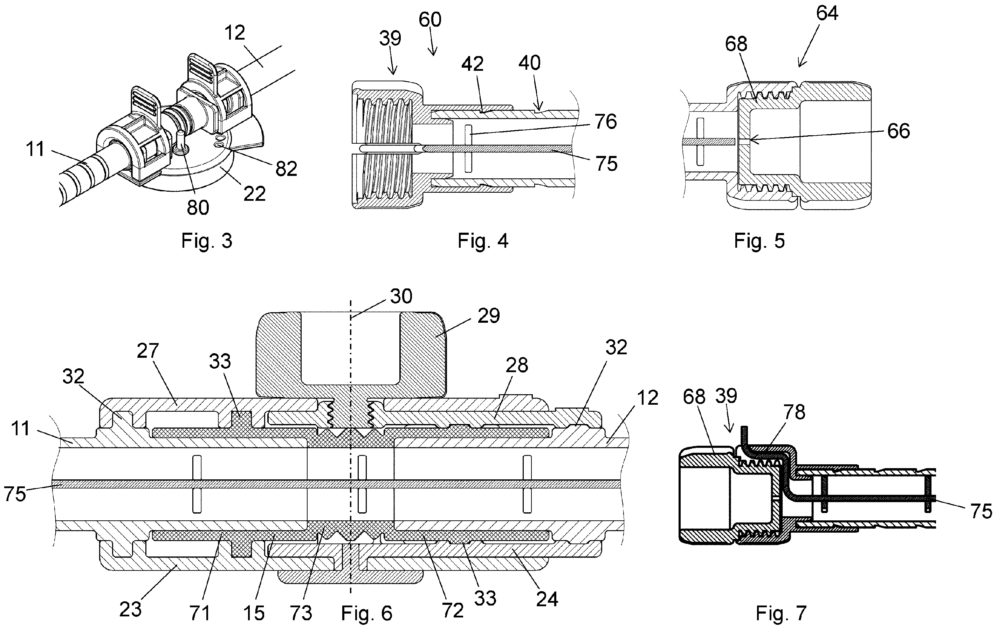

Das erste Rohr 11 ist hinsichtlich seiner Länge einstellbar. Dies ist hier beispielhaft so gelöst, dass das erste Rohr 11 entlang seiner Längsrichtung in typischerweise regelmäßigen Abständen Formelemente 40 aufweist und ein Abschlusselement 39 korrespondierende Rastelemente 42 aufweist. Das erste Rohr 11 kann also an einer beliebigen, gewünschten Position gekürzt werden, beispielsweise durch Schneiden. Im Anschluss kann das Abschlusselement 39 aufgesteckt werden, wobei ein oder mehrere Rastelemente 42 des Abschlusselements 39 mit einem oder mehreren Formelementen 40 des ersten Rohrs 11 verrasten. Auf diese Weise wird ein axiales Abziehen des Abschlusselements 39 durch Formschluss verhindert. Dies ist als vergrößertes Detail in Figur 4 dargestellt, welche zusätzlich ein zentral im ersten Rohr 11 liegendes Inlay 75 sowie Abstandhalter 76 zum Halten des Inlays 75 in der zentralen Position zeigt.

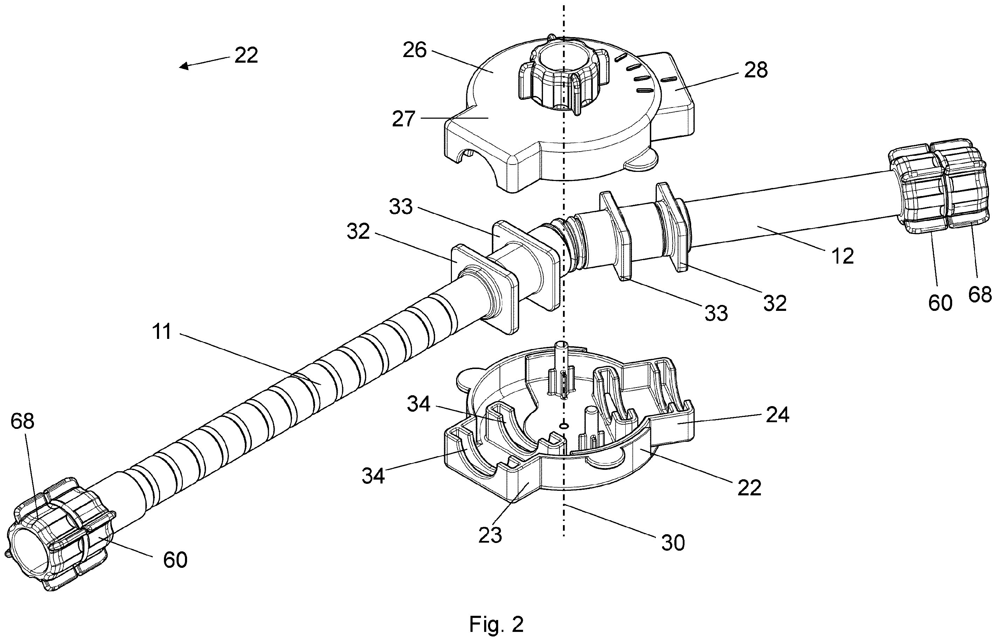

Figur 2 zeigt eine ähnliche Darstellung in Figur 1, bei der die einzelnen Teile des Gehäuses 20 nach Art einer Explosionszeichnung dargestellt sind. Auf diese Weise ist das mittig zwischen dem ersten Rohr 11 und dem zweiten Rohr 12 befindliche Verbindungsstück 15 sichtbar, welches die Innenräume der beiden Rohre 11, 12 strömungstechnisch miteinander verbindet. Das Verbindungsstück 15 weist ebenfalls eine rohrartige Grundform auf und ist aus einem flexiblen, beispielsweise gummielastischen Material hergestellt. So sind in einem vorgegebenen Winkelbereich von etwa 0° bis 40° beliebige Winkel zwischen dem ersten Rohr 11 und dem zweiten Rohr 12 einstellbar.

In seinem jeweils zum Verbindungsstück 15 weisenden Bereich weist sowohl das erste Rohr 11 als auch das zweite Rohr 12 ein Formschlusselement 32 in Form einer umlaufenden Scheibe auf. Das Verbindungstück 15 weist ebenfalls an jeder seiner Seiten ein derartiges Formschlusselement 33 auf.

Es ist ersichtlich, dass das Gehäuseunterteil 22 einen ersten Gehäuseunterteilabschnitt 23, der das erste Rohr 11 aufnimmt, sowie einen zweiten Gehäuseunterteilabschnitt 24 umfasst, welcher das zweite Rohr 12 aufnimmt. Der erste Gehäuseunterteilabschnitt 23 der zweite Gehäuseunterteilabschnitt 24 sind um eine gemeinsame Drehachse 30 relativ zueinander drehbar. Analog dazu umfasst das Gehäuseoberteil 26 einen ersten Gehäuseoberteilabschnitt 27 und einen um dieselbe Drehachse 30 relativ dazu drehbaren zweiten Gehäuseoberteilabschnitt 28.

Gehäuseunterteil 22 und Gehäuseoberteil 26 können typischerweise je nach Anforderung miteinander verbunden oder, wie dargestellt, voneinander getrennt werden. Dies ist bevorzugt manuell und zerstörungsfrei möglich. Es ist ersichtlich, dass im hier gezeigten Beispiel das Gehäuseunterteil 22 nach oben weisende Stifte umfasst, die in entsprechende Öffnungen im Gehäuseoberteil 26 eingesteckt werden können, um das Gehäuse 20 zu schließen. Es sind jedoch beliebige andere Verschlussmöglichkeiten möglich.

Im Gehäuseunterteil 22 und/oder im Gehäuseoberteil 26 sind Aussparungen 34 zur Aufnahme der Formschlusselemente 32, 33 vorhanden. Wird die Einheit aus erstem Rohr 11, Verbindungsstück 15 und zweitem Rohr 12 in das Gehäuseunterteil 22 gelegt und insbesondere mit dem Gehäuseoberteil 26 verschlossen, kann weder die Verbindung zwischen dem ersten Rohr 11 und dem Verbindungsstück 15 noch die Verbindung aus dem Verbindungsstück 15 und dem zweiten Rohr 12 gelöst werden.

Figur 6 zeigt eine Schnittzeichnung einer derartigen Verbindung zwischen dem ersten Rohr 11, dem Verbindungsstück 15 sowie dem zweiten Rohr 12. Hier ist gut erkennbar, dass auf der linken Seite der erste Gehäuseunterteilabschnitt 23 und der erste Gehäuseoberteilabschnitt 27 sowohl das erste Rohr 11 - siehe Formschlusselemente 32 - als auch den linken Teil des Verbindungsstücks 15 - siehe Formschlusselemente 33 - hält. Auf der rechten Seite werden der rechte Teil des Verbindungsstücks 15 sowie das zweite Rohr 12 hingegen vom zweiten Gehäuseoberteilabschnitt 28 und vom zweiten Gehäuseunterteilabschnitt 24 gehalten. Ferner ist sichtbar, dass der zweite Gehäuseunterteilabschnitt 24 und der zweite Gehäuseoberteilabschnitt 28 auf der rechten Seite vom ersten Gehäuseunterteilabschnitt 23 sowie vom ersten Gehäuseoberteilabschnitt 27 umgeben sind. Bei einer relativen Drehung gleitet der erste Gehäuseoberteilabschnitt 27 relativ zum zweiten Gehäuseoberteilabschnitt 28 und es gleitet der erste Gehäuseunterteilabschnitt ein 20 relativ zum zweiten Gehäuseunterteilabschnitt 24.

Zudem ist eine Sperreinrichtung 29 dargestellt, mit welcher die Drehung der zwei Gehäuseunterteilabschnitte 23, 24 sowie die Drehung der zwei Gehäuseoberteilabschnitte 27, 28 sind die gesperrt werden kann. Die Sperrrichtung 29 ist hier beispielhaft ausgeführt als entlang der Drehachse 30 ausgerichtete Schraube, die in ein Gewinde im zweiten Gehäuseoberteilabschnitt 28 eingeschraubt werden kann und in der Folge den ersten Gehäuseoberteilabschnitt 27 zwischen dem zweiten Gehäuseoberteilabschnitt 28 und dem Kopf der Schraube eingeklemmt, sodass ein Gleiten nicht mehr möglich ist und der Winkel zwischen dem ersten Rohr 11 und dem zweiten Rohr 12 folglich fixiert ist.

Figur 6 zeigt ferner, dass das Verbindungsstück 15 einen ersten Anschlussbereich 71 zum Aufstecken auf das erste Rohr 11, einen zweiten Anschlussbereich 72 zum Aufstecken auf das zweite Rohr 12 und einen dazwischen liegenden mittleren Bereich 73 aufweist. Der Innendurchmesser des Verbindungsstücks 15 ist im mittleren Bereich 73 kleiner als im ersten und zweiten Anschlussbereich 71, 72, und zwar so, dass der mittlere Bereich 73 denselben Innendurchmesser aufweist wie der angrenzende Bereich des ersten Rohrs 11 und des zweiten Rohrs 12.

Figur 3 zeigt eine alternative Möglichkeit, den Winkel zu fixieren, also eine Alternative Sperreinrichtung. Hierbei sind Öffnungen 82 im ersten Gehäuseunterteilabschnitt sowie im zweiten Gehäuseunterteilabschnitt angeordnet und es mindestens ein Stift ähnlich dem dargestellten Stift 80 vorhanden, der in die Öffnungen eingesteckt werden kann. Der Stift verläuft somit durch die Öffnung in einem der Gehäuseunterteilabschnitte hindurch und in die Öffnung in dem darunter befindlichen Gehäuseunterteilabschnitt hinein, sodass eine relative Rotation der beiden Gehäuseunterteilabschnitte nicht mehr möglich ist.

Figur 5 zeigt ein vergrößertes Detail des zweiten Endstücks 64, in dem ersichtlich ist, dass sich in der Verschlusskappe 68 eine beispielhaft radial und mittig angeordnete Entlüftungsöffnung 66 befindet, um während des Einpressens der Gussmasse verdrängte Luft ausströmen zu lassen.

Figur 7 zeigt ein vergrößertes Detail, beispielsweise des ersten Endstücks, in dem ersichtlich ist, wie ein metallisches Inlay 75 im Bereich eines Abschlusselements 39 gehalten werden kann. Im oben dargestellten Bereich des Gewindes, beispielsweise des Verbindungsbereichs 62, ist eine Aussparung 78 in Form einer parallel zu Längsachse des betreffenden Rohrs verlaufenden Nut vorhanden. Das Inlay 75, beispielsweise ein Kirschner-Draht, wird nun vor dem Einschrauben der Verschlusskappe 68 in die Aussparung 78 gelegt und anschließend wird die Verschlusskappe 68 aufgeschraubt.

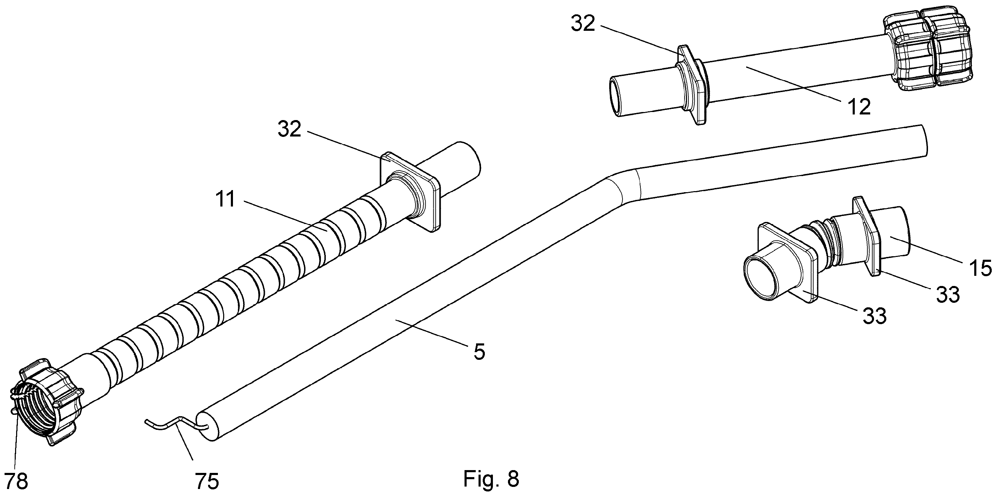

Figur 8 zeigt eine Zusammenschau des ersten Rohrs 11, des zweiten Rohrs 12, des Verbindungsstücks 15 sowie eines hergestellten Marknagelspacers 5 nach dem Entformen. Nach dem Entfernen des Gehäuses können sowohl das erste Rohr 11 als auch das zweite Rohr 12 typischerweise jeweils nach außen abgezogen werden. Das Verbindungsstück 15 kann anschließend ebenfalls abgezogen oder beispielsweise durch Aufschneiden entfernt werden. Der resultierende Marknagelspacer 5 mit dem zuvor eingestellten Winkel ist nun fertiggestellt.

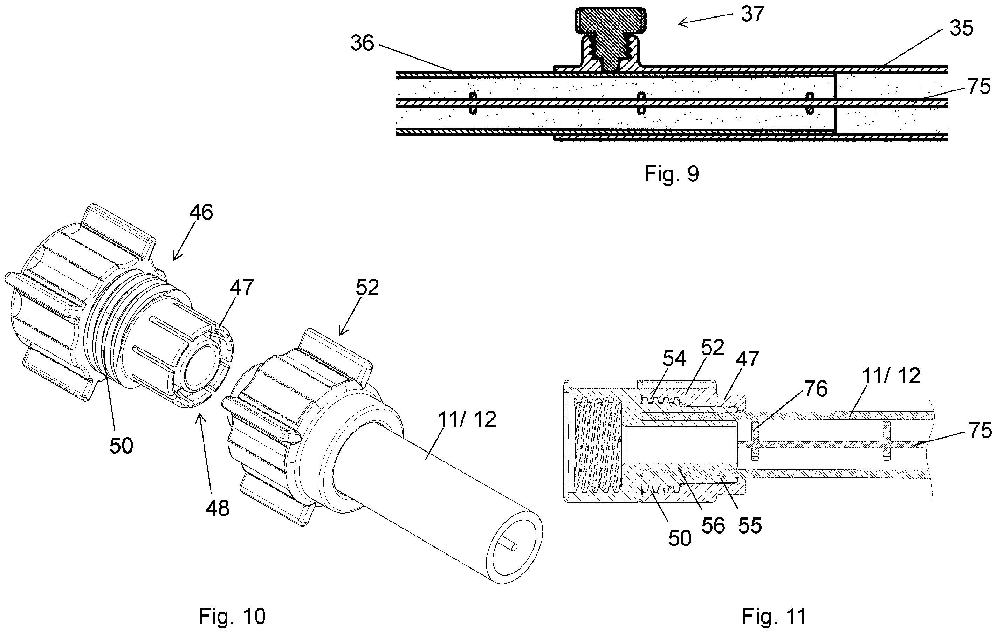

Figur 9 zeigt ein weiteres Detail, bei dem mindestens eines der Rohre 11, 12 teleskopartig ausziehbar ist. Das jeweilige Rohr umfasst ein äußeres Rohr 35 sowie ein inneres Rohr 36, welches in dem äußeren Rohr 35 axial verschieblich ist. Bevorzugt ist zum Befestigen des äußeren Rohrs 35 an dem inneren Rohr 36 eine Befestigungseinrichtung 37 vorhanden. Im hier gezeigten Beispiel umfasst diese eine Schraube, die in ein Gewinde am äußeren Rohr 35 beispielsweise senkrecht zur Längsachse des Rohrs eingeschraubt werden kann und in der Folge von außen auf das innere Rohr 36 drückt, um auf diese Weise eine kraftschlüssige Verbindung herzustellen.

Die Figuren 10 und 11 zeigen vergrößerte Details einer weiteren Alternative zu Längeneinstellung eines oder beide Rohre 11, 12. Es ist ersichtlich, dass das Abschlusselement 39 hier ein links dargestelltes Klemmteil 46 mit einem Klemmbereich 48 umfasst, in welchen das Rohr 11 oder 12 in axialer Richtung eingesteckt werden kann. Der Klemmbereich 48 ist definiert durch hier beispielhaft außen liegende, flexible Klemmelemente 47 sowie ein innenliegendes gegenüberliegendes Teil 56. Die Klemmelemente 47 sind beispielsweise durch axiales Schlitzen eines äußeren, rohrförmigen Abschnitts des Klemmteils 46 hergestellt worden. Das gegenüberliegende Teil 56 ist beispielsweise ein innerer, rohrförmiger Abschnitt des Klemmteil 46. Das Klemmteil 46 umfasst ferner ein erstes Gewinde 50.

Das Abschlusselement 39 umfasst ferner eine Überwurfmutter 52, die über das jeweilige Rohr 11 oder 12 gestülpt ist und ein mit dem ersten Gewinde 50 korrespondierendes zweites Gewinde 54 umfasst. Wird die Überwurfmutter 52 nun auf das Klemmteil 46 geschraubt, werden die Klemmelemente 47 axial nach innen gepresst und klemmen damit das jeweiligen Rohr 11 oder 12 gegen das gegenüberliegende Teil 56. Folglich ist das Abschlusselement 39 kraftschlüssig mit dem Rohr 11 oder 12 verbunden. Es können zudem radial nach innen ragende Klemmvorsprünge 55 an einem oder mehreren Klemmelementen 57 vorhanden sein, wie in Figur 11 gezeigt, die je nach Ausgestaltung und Eigenschaften der Materialien eine kraftschlüssige und/oder formschlüssige Verbindung ermöglichen. Alternativ oder ergänzend kann der Klemmbereich 48 konisch ausgeführt sein, um die Verbindung zur ermöglichen und/oder zu verbessern.

**Bezugszeichenliste**

| | |
|---|---|
| Marknagelspacer | 5 |
| Vorrichtung | 10 |
| Erstes Rohr | 11 |
| Zweites Rohr | 12 |
| Verbindungsstück | 15 |
| Fixierungseinrichtung | 18 |
| Gehäuse | 20 |
| Gehäuseunterteil | 22 |
| erster Gehäuseunterteilabschnitt | 23 |
| zweiter Gehäuseunterteilabschnitt | 24 |
| Gehäuseoberteil | 26 |
| erster Gehäuseoberteilabschnitt | 27 |
| zweiter Gehäuseoberteilabschnitt | 28 |
| Sperreinrichtung | 29 |
| Drehachse | 30 |
| Formschlusselement | 32 |
| Formschlusselement | 33 |
| Aussparung | 34 |
| Äußeres Rohr | 35 |
| Inneres Rohr | 36 |
| Befestigungseinrichtung | 37 |
| Abschlusselement | 39 |
| Formelemente | 40 |
| Rastelemente | 42 |
| Klemmteil | 46 |
| Klemmelement | 47 |
| Klemmbereich | 48 |
| Erstes Gewinde | 50 |
| Überwurfmutter | 52 |
| Zweites Gewinde | 54 |
| Klemmvorsprung | 55 |
| Gegenüberliegendes Teil | 56 |
| Gussmassequelle | 59 |
| Erstes Endstück | 60 |
| Verbindungsbereich | 62 |
| Zweites Endstück | 64 |
| Entlüftungsöffnung | 66 |
| Verschlusskappe | 68 |
| Erster Anschlussbereich | 71 |
| Zweiter Anschlussbereich | 72 |
| Mittlerer Bereich | 73 |
| Inlay | 75 |
| Abstandhalter | 76 |
| Aussparung | 78 |
| Stift | 80 |
| Öffnung | 82 |

## Patentansprüche

1. Vorrichtung (10) zur Herstellung eines Marknagelspacers (5), umfassend ein erstes Rohr (11), ein zweites Rohr (12) und ein flexibles Verbindungsstück (15) zum strömungstechnischen Verbinden des ersten Rohrs (11) mit dem zweiten Rohr (12), wobei das erste Rohr (11) und das zweite Rohr (12) in unterschiedlichen Winkeln zueinander positionierbar sind.

2. Vorrichtung (10) nach dem vorhergehenden Anspruch, wobei die Vorrichtung (10) ferner eine Fixierungseinrichtung (18) zur Fixierung eines Winkels zwischen dem ersten Rohr (11) und dem zweiten Rohr (12) aufweist.

3. Vorrichtung (10) nach dem vorhergehenden Anspruch, wobei die Vorrichtung (10) ein Gehäuseunterteil (22) aufweist, welches einen ersten Gehäuseunterteilabschnitt (23) und einen zweiten Gehäuseunterteilabschnitt (24) umfasst, wobei eine relative Drehung der zwei Gehäuseunterteilabschnitte (23, 24) um eine Drehachse (30) möglich ist, wobei der erste Gehäuseunterteilabschnitt (23) zum Halten des ersten Rohrs (11) eingerichtet ist und der zweite Gehäuseunterteilabschnitt (24) zum Halten des zweiten Rohrs (12) eingerichtet ist, wobei insbesondere eine Sperreinrichtung (29) vorhanden ist, um eine Drehung der zwei Gehäuseunterteilabschnitte (23, 24) selektiv zu sperren.

4. Vorrichtung (10) nach dem vorhergehenden Anspruch, wobei die Vorrichtung (10) ein Gehäuseoberteil (26) aufweist, welches einen ersten Gehäuseoberteilabschnitt (27) und einen zweiten Gehäuseoberteilabschnitt (28) umfasst, wobei eine relative Drehung der zwei Gehäuseoberteilabschnitte (27, 28) um die Drehachse (30) möglich ist, wobei das Gehäuseoberteil (26) mit dem Gehäuseunterteil (22) verbunden oder verbindbar ist, um gemeinsam ein Gehäuse (20) zu bilden, wobei insbesondere eine Sperreinrichtung (29) vorhanden ist, um eine Drehung der zwei Gehäuseoberteilabschnitte (27, 28) selektiv zu sperren.

5. Vorrichtung (10) nach einem der Ansprüche 2 bis 4, wobei das erste Rohr (11) und/oder das zweite Rohr (12) ein nach außen ragendes Formschlusselement (32) aufweist, welches derart gehalten ist, dass ein axiales Herausziehen des jeweiligen Rohrs (11, 12) blockiert ist.

6. Vorrichtung (10) nach einem der vorhergehenden Ansprüche, wobei eine Länge des ersten Rohrs (11) und/oder eine Länge des zweiten Rohrs (12) einstellbar ist.

7. Vorrichtung (10) nach dem vorhergehenden Anspruch, wobei das erste Rohr (11) und/oder das zweite Rohr (12) für eine Längeneinstellung teleskopartig ausgebildet ist.

8. Vorrichtung (10) nach einem der Ansprüche 6 und 7, wobei das erste Rohr (11) und/oder das zweite Rohr (12) zum Einstellen der Länge gekürzt werden kann und die Vorrichtung (10) ein Abschlusselement (39) zum Anordnen an der gekürzten Seite aufweist.

9. Vorrichtung (10) nach dem vorhergehenden Anspruch, wobei das erste Rohr (11) und/oder das zweite Rohr (12) an seiner Außenseite Formelemente (40) zur Herstellung einer Rastverbindung aufweist, welche in Abständen entlang der Längsachse des Rohrs (11, 12) angeordnet sind, und das Abschlusselement (39) ein oder mehrere Rastelemente (42) aufweist, die mit den jeweiligen Formelementen (40) verrastet werden können.

10. Vorrichtung (10) nach einem der Ansprüche 8 und 9, wobei das Abschlusselement (39) umfasst:
- ein Klemmteil (46) mit einem Klemmbereich (48) zum Einklemmen des gekürzten Rohrs (11, 12), wobei das Klemmteil (46) ein erstes Gewinde (50) aufweist, sowie
- eine auf das Rohr (11, 12) aufsteckbare Überwurfmutter (52) mit einem zweiten Gewinde (54),
wobei das gekürzte Rohr (11, 12) durch Aufschrauben der Überwurfmutter (52) auf das Klemmteil (46) im Klemmbereich (48) festgeklemmt werden kann.

11. Vorrichtung (10) nach einem der vorhergehenden Ansprüche, ferner umfassend:
- ein erstes Endstück (60) zur Anordnung an einem dem Verbindungsstück (15) abgewandten Ende des ersten Rohrs (11) oder des zweiten Rohrs (12), wobei an einer dem ersten Rohr (11) bzw. dem zweiten Rohr (12) abgewandten Seite des ersten Endstücks (60) ein Verbindungsbereich (62) zum Verbinden mit einer Gussmassequelle (59) angeordnet ist, und/oder
- ein zweites Endstück (64) zur Anordnung an einem dem Verbindungsstück (15) abgewandten Ende des ersten Rohrs (11) oder des zweiten Rohrs (12), wobei im zweiten Endstück (64) eine Entlüftungsöffnung (66) angeordnet ist, die das Rohr (11, 12) mit der Umgebung verbindet.

12. Vorrichtung (10) nach einem der vorhergehenden Ansprüche, wobei das Verbindungsstück (15) einen ersten Anschlussbereich (71) zum Aufstecken auf das erste Rohr (11), einen zweiten Anschlussbereich (72) zum Aufstecken auf das zweite Rohr (12) und einen zwischen den Anschlussbereichen (71, 72) befindlichen mittleren Bereich (73) aufweist, wobei ein innerer Querschnitt des Verbindungsstücks (15) im mittleren Bereich (73) kleiner ist als im ersten und zweiten Anschlussbereich (71, 72).

13. Vorrichtung (10) nach einem der vorhergehenden Ansprüche, ferner umfassend ein in dem ersten Rohr (11) und/oder dem zweiten Rohr (12) angeordnetes Inlay (75) zum inneren Verstärken des herzustellenden Marknagelspacers (5).

14. Verwendung einer Gussform zur Herstellung eines Marknagelspacers (5).

15. Verfahren zur Herstellung eines Marknagelspacers (5) unter Verwendung einer Vorrichtung (10), wobei die Vorrichtung (10) ein erstes Rohr (11), ein zweites Rohr (12) und ein flexibles Verbindungsstück (15) zum strömungstechnischen Verbinden des ersten Rohrs (11) mit dem zweiten Rohr (12) umfasst, wobei das Verfahren umfasst:
- relatives Positionieren des ersten Rohrs (11) und des zweiten Rohr (12) in einem gewünschten Winkel zueinander, sowie
- Einfüllen einer Gussmasse in ein erstes Rohr (11), das zweite Rohr (12) und das Verbindungsstück (15) zur Herstellung des Marknagelspacers (5).
